Europäisches Patentamt

European Patent Office

Office européen des brevets

(19)

(11) EP 0 734 749 B1

(12) EUROPÄISCHE PATENTSCHRIFT

(45) Veröffentlichungstag und Bekanntmachung des Hinweises auf die Patenterteilung:
25.09.2002 Patentblatt 2002/39

(51) Int Cl.⁷: **B01D 15/00**, G01N 33/543, B01J 20/28

(21) Anmeldenummer: 96104919.4

(22) Anmeldetag: 28.03.1996

(54) **Verfahren zur Isolierung eines biologischen Materials**

Process for isolating a biological material

Procédé pour séparer un produit biologique

(84) Benannte Vertragsstaaten:
**CH DE ES FR GB IT LI NL**

(30) Priorität: **01.04.1995 DE 19512361**

(43) Veröffentlichungstag der Anmeldung:
**02.10.1996 Patentblatt 1996/40**

(73) Patentinhaber: **Roche Diagnostics GmbH
68298 Mannheim (DE)**

(72) Erfinder:
• **Bienhaus, Gerhard, Dr.
82407 Wielenbach (DE)**
• **Fritz, Michael
68647 Biblis (DE)**
• **Schwab, Jürgen
68775 Ketsch (DE)**
• **Geisler, Edda
68199 Mannheim (DE)**
• **Harttig, Herbert, Dr.
67122 Altrip (DE)**
• **Macho, Heinz
64658 Fürth (DE)**

(56) Entgegenhaltungen:
WO-A-94/29721      US-A- 4 021 352
US-A- 4 644 807     US-A- 4 909 992

## Beschreibung

Gegenstand der Erfindung ist ein Verfahren zur Isolierung eines biologischen Materials durch Bindung des biologischen Materials an eine feste Phase und Freisetzung dieses Materials durch ein spezielles Vorgehen sowie ein zur Isolierung des biologischen Materials geeignetes System.

In jüngerer Zeit finden biologische Materialien auf vielen Gebieten besonderes Interesse. Dies wird insbesondere dadurch erleichtert, daß seit wenigen Jahrzehnten Möglichkeiten bestehen, biologische Materialien von anderen Materialien abzutrennen. Im allgemeinen liegen biologische Materialien nämlich in einem komplexen Gemisch mit anderen Materialien vor. Die meisten biologischen Materialien liegen darüber hinaus auch in sehr geringen Mengen, verglichen mit den übrigen Bestandteilen des biologischen Individuums, vor. Veränderungen von biologischen Materialien gegenüber einem Normalzustand können dazu benutzt werden, um den Zustand eines biologischen Individuums zu diagnostizieren. Verfahren zur Analyse von biologischen Materialien finden daher insbesondere auf dem Gebiet der Molekularbiologie und dem Gesundheitswesen weite Verbreitung. Je nach Ziel des Analyseverfahrens wird ein mehr oder weniger spezifisches Isolierungsverfahren gewählt.

Isolierungsverfahren für biologische Materialien gibt es in verschiedener Art, abhängig von dem zu isolierenden biologischen Material und dessen anschließender Verwendung. In der Analyse von Antigenen und Antikörpern oft verwendeten Isolierungsart wird das biologische Material, z. B. ein Antigen, Antikörper oder eine Nukleinsäure, an die nicht poröse Innenwand einer Küvette aus Glas aus Polystyrol gebunden. Die Bindung des biologischen Materials ist in diesem Fall meist so spezifisch, daß nur das später nachzuweisende biologische Material an der Oberfläche immobilisiert wird. In diesen Verfahren ist eine erneute Freisetzung des biologischen Materials nicht angestrebt und sogar für eine nachfolgende quantitative Bestimmung schädlich.

Eine zweite Art von Verfahren benutzt quellbare, poröse Materialien zur Auftrennung biologischer Materialien, z. B. nach ihrem Molekulargewicht. In diesen Verfahren findet eine Bindung des biologischen Materials an eine feste Phase nicht statt. Die Auftrennung findet im wesentlichen unter Ausnutzung des unterschiedlichen Durchdringungsverhaltens von biologischen Materialien aufgrund unterschiedlicher Größe statt.

Eine dritte Art von Isolierungsverfahren beruht auf der unterschiedlich starken Bindung unterschiedlicher biologischer Materialien an porösen Materialien, die durch Anbringung spezifitätsvermittelnder Gruppen eine besondere Affinität zu dem biologischen Material aufweisen. Hierzu werden säulenartig aufgebaute Schüttungen von körnigem Affinitätsmaterial verwendet. Eine Flüssigkeit, die das biologische Material enthält, wird zum Durchtritt durch diese Schüttung veranlaßt. Das zu isolierende biologische Material wird an der Oberfläche der porösen Partikel an fiir das biologische Material affinen Gruppen gebunden, während die übrigen Inhaltsstoffe mit der Flüssigkeit aus der Säule austreten können. In einem anschließenden Schritt wird das biologische Material von dem porösen Material freigesetzt, indem eine die Bindung zur porösen Matrix auflösende Elutionsflüssigkeit in derselben Fließrichtung durch die Säule geleitet wird. Das biologische Material befindet sich anschließend in der Elutionsflüssigkeit.

Die Elutionsflüssigkeit wurde in den bisher verwendeten Verfahren entweder durch Anlegen von Druck in Laufrichtung der Flüssigkeit oder Unterdruck auf der Seite des Flüssigkeitsaustritts oder jedoch durch Zentrifugation der Säule, wodurch die Elutionsflüssigkeit aus der porösen Matrix herausgeschleudert wurde, durch die poröse Matrix befördert. Dieses Verfahren verwenden jedoch Vakuumpumpen oder Zentrifugatoren, also Geräte, die in der medizinischen Routinediagnostik oft nur für diese einzige Einsatzart Verwendung finden. Darüber hinaus ist ihr Einsatz, insbesondere der Zentrifugatoren, zeitaufwendig und erlaubt keine kontinuierliche Abarbeitung von Analysen.

Aufgabe der vorliegenden Erfindung war die Bereitstellung eines neuen Isolierungsverfahrens für biologische Materialien, welches im Hinblick auf den Stand der Technik Verbesserungen mit sich bringt.

Gegenstand der Erfindung ist ein Verfahren zur Isolierung eines biologischen Materials durch Bereitstellung von an eine komprimierbare poröse Matrix gebundenem biologischen Material und Kompression der Matrix sowie unter Bedingungen, bei denen das biologische Material von der Oberfläche der Matrix in eine Elutionsflüssigkeit freigesetzt wird. Ebenfalls Gegenstand der Erfindung ist ein System zur Isolierung des biologischen Materials.

Unter Isolierungsverfahren gemäß der vorliegenden Erfindung werden Verfahren verstanden, bei denen ein oder mehrere Bestandteile eines Gemisches von den übrigen Bestandteilen dieses Gemisches abgetrennt wird. Insbesondere werden darunter solche Verfahren verstanden, bei denen der oder die abzutrennenden Bestandteile an eine feste Phase gebunden werden, die übrige Flüssigkeit entfernt werden, und der oder die gebundenen Bestandteile anschließend in eine andere Flüssigkeit freigesetzt werden.

Unter einem biologischen Material werden solche organischen Verbindungen verstanden, die in einer Beziehung zu Lebewesen, wie Tieren, Menschen, Viren, Bakterien oder Pflanzen, stehen. Hierzu gehören beispielsweise die Inhaltsstoffe von Lebewesen. Besonders bevorzugte Inhaltsstoffe sind solche, die entweder in gelöster Form vorliegen, oder die in einer Flüssigkeit gelöste Form gebracht werden können, die jedoch auch an eine feste Matrix gebunden werden können. Hierzu gehören einerseits niedermolekulare Substanzen (mit einem Molekulargewicht von weniger als 2.000 D), wie Vitamine, aber auch therapeutisch wirksame Substanzen und Hormone, aber auch hochmolekulare Substanzen (mit einem Molekulargewicht von mehr als 2.000 D), wie Biopolymere, die aus Monomereinheiten aufgebaut sind. Zu diesen zählen beispielsweise die Proteine und Nukleinsäuren. Das erfindungsgemäße Verfahren ist insbesondere zur Isolierung von Nukleinsäuren geeignet. Bei den Proteinen sind insbesondere die immunologisch aktiven Substanzen bevorzugt, wie Antigene und Antikörper.

Biologische Materialien, deren Isolierung besonders bevorzugt möglich ist, werden aus einer aus dem Lebewesen gewonnen Flüssigkeit an die Matrix gebunden. Je nach Teil des Lebewesens, aus welchem das biologische Material isoliert werden soll, ist eine Vorbehandlung des Lebewesens erforderlich. Eine solche Vorbereitung kann dazu dienen, das biologische Material aus dem Lebewesen freizusetzen. Eine solche Vorbereitung findet zum Beispiel statt, wenn es sich bei dem Lebewesen um ein Bakterium oder um eine Gruppe von Bakterien handelt. In diesem Fall wird vorzugsweise eine Zerstörung der Zellen vorgenommen, wodurch die Inhaltsstoffe des Lebewesens in die Flüssigkeit austreten können. Anschließend kann eine Abtrennung der ggf. gebildeten Feststoffe stattfinden. Befindet sich das biologische Material schon in zugänglicher Form in einer Flüssigkeit, z. B. einer Körperflüssigkeit, wird in vielen Fällen noch eine Abtrennung von Zellen oder anderen, die Isolierung des biologischen Materials störenden Stoffen vorgenommen. Dies kann beispielsweise durch Filtration oder an Affinitätsmaterialien geschehen. Ergebnis der Probenvorbereitung ist in jedem Fall eine Probenflüssigkeit, die das biologische Material in einer Form enthält, in der es an eine Matrix gebunden werden kann.

In einem Schritt des erfindungsgemäßen Verfahrens wird das biologische Material in an eine komprimierbare poröse Matrix gebundene Form bereitgestellt. Unter einer Matrix im Sinne der Erfindung wird ein in der Flüssigkeit, in der sich das biologische Material befindet, nicht lösliches Material verstanden. Die chemische Zusammensetzung der Matrix wird dadurch bestimmt, daß die Matrix komprimierbar und porös sein muß. Aus diesem Grund kann es sich um organische oder anorganische Polymere handeln. Unter organischen Polymeren werden beispielsweise die Kunststoffe, z. B. Polystyrol, aber auch Cellulose, z. B. Papier, verstanden. Unter anorganischen Polymeren werden insbesondere Stoffe verstanden, die einen Glasanteil haben. Die Matrix kann jedoch auch aus einem Metall aufgebaut sein.

Die erfindungsgemäße komprimierbare poröse Matrix stellt ein räumlich ausgedehntes Gebilde dar, welches einen unlöslichen Anteil aus dem oben genannten Material und einen durch Flüssigkeit auffüllbaren Anteil aufweist. Dieser auffüllbare Anteil wird im folgenden auch als Innenvolumen bezeichnet. Das Innenvolumen dehnt sich zwischen dem unlöslichen Anteil aus. Hierdurch bildet es ein System aus zusammenhängenden Poren bzw. Leerräumen. Man könnte dieses System auch als bevorzugt offenporig bezeichnen.

Der unlösliche Anteil bildet in diesem System ebenfalls ein räumlich ausgedehntes Gebilde, welches bevorzugt ebenfalls zusammenhängend ist. Möglich, aber nicht bevorzugt, ist der Fall, daß die Matrix ein mehrkomponentiges System darstellt, z. B. ein System, bei dem eine partikuläre Komponente zwischen zwei partikuläre Komponenten in Richtung auf den Innenraum (C14) bzw. in Richtung der unteren Öffnung im Formkörper festhaltende flüssigkeitsdurchlässige Komponenten, z. B. Vliese, eingesiegelt ist. Die partikuläre Komponente kann dann z. B. eine Aufschlämmung zwischen den beiden Komponenten bilden; zur Gewinnung der Flüssigkeit werden die beiden Komponenten aufeinander zu geschoben, wodurch die Aufschlämmung verdichtet und das darin enthaltende Flüssigkeitsvolumen reduziert wird.

Beispiele für eine solche komprimierbare poröse Matrix sind schwammartige Gebilde und Gebilde, die aus Fasern aufgebaut sind, z. B. Vliese. Bevorzugt ist die Matrix bezüglich der Flüssigkeit saugfähig und an ihrer Oberfläche durch die Probenflüssigkeit benetzbar.

Als komprimierbar wird eine Matrix im Sinne der Erfindung dann bezeichnet, wenn durch Zusammendrücken des räumlich ausgedehnten Gebildetes aus unlöslichem Anteil und Innenvolumen das Innenvolumen um 50 % oder mehr, bevorzugt 70 % oder mehr, besonders bevorzugt 90 % oder mehr, verkleinert werden kann. Ein vollständiges Verschwinden des Innenvolumens wird aus praktischen Gründen wahrscheinlich nicht erreicht werden können, weshalb im allgemeinen ein gewisser Anteil des Innenvolumens und somit von Flüssigkeit in der Matrix verbleiben wird.

Der Volumenanteil des unlöslichen Materials zu dem Innenvolumen liegt bevorzugt in einem Bereich zwischen 10:1 und 1:100, besonders bevorzugt zwischen 1:1 und 1:50.

Die Komprimierbarkeit der porösen Matrix muß nicht unbedingt reversibel sein, d. h., wenn der Druck auf die Matrix entfernt wird, muß diese Matrix nicht unbedingt wieder ihre ursprüngliche Form annehmen. Die Ausdehnung der Matrix hängt u. a. von der Menge an zu bindendem biologischen Material ab. Die äußere Form kann beliebig sein. Sie richtet sich u. a. nach der Art der Immobilisierung des biologischen Materials und der darauffolgenden Freisetzung. Bevorzugt hat die Matrix die Form eines Plättchens.

Das unlösliche Material der Matrix kann als solches schon so gewählt werden, daß das biologische Material eine Affinität zu dem Material hat, sodaß eine Bindung stattfindet. Ein solcher Fall ist gegeben für die Immobilisierung von Nukleinsäuren, wenn als unlösliches Material ein Glasfaservlies verwendet wird. Es ist nämlich bekannt, daß alle Nukleinsäuren eine gewisse Affinität zu Glasoberflächen aufweisen. Wenn das Material selbst keine Affinität zu dem zu isolierenden biologischen Material aufweist, kann dieses Material an seine Oberfläche modifiziert werden, sodaß eine Bindung stattfindet. Für den Fall, daß Nukleinsäuren gebunden werden sollen, können hierzu beispielsweise sogenannte Fangsonden, d. h. Nukleinsäuren, die eine zu der zu isolierenden Nukleinsäure komplementäre Sequenz aufweisen, an die Oberfläche gebunden sein. Für den Fall der Isolierung von immunologisch reaktiven Verbindungen, wie z. B. Antigenen oder Antikörpern, können die entsprechenden Immunpartner immobilisiert werden, d. h. Antikörper bzw. Antigene. Für die Bindung der Fangsonden bzw. immunreaktiven Verbindungen stehen die Verfahren zur Verfügung, die auch für nicht poröse Materialien bekannt sind.

Die Bereitstellung des an die komprimierbare, poröse, Matrix gebundenen biologischen Materials kann beispielsweise dadurch geschehen, daß die Matrix mit einer Lösung des biologischen Materials in Kontakt gebracht und für eine ausreichende Zeit inkubiert wird. Da im allgemeinen Fall die Menge an Lösung, in der das biologische Material vorliegt, größer sein wird als das Innenvolumen der Matrix, ist es bevorzugt, die Lösung durch die Matrix hindurchzuführen, z. B. durch Absaugen oder Zentrifugation. Besonders bevorzugt wird die Lösung jedoch in einem Gefäß durch die Matrix hindurch gedrückt, ohne daß ein Unterdruck angewendet werden muß und ein Zentrifugationsschritt erforderlich ist. Bevorzugt wird anschließend die im Innenraum der Matrix vorliegende Flüssigkeit, welche nicht zu bindende Bestandteile der Lösung enthält, aus der Matrix entfernt, z. B. ebenfalls durch Absaugen, Zentrifugieren oder Auspressen. Gewünschtenfalls kann die Matrix, welche nun das biologische Material gebunden enthält, gewaschen und von eventuellen anhaftenden Verunreinigungen befreit werden.

Der Kern der Erfindung liegt in der Art der Freisetzung des biologischen Materials aus der Matrix. Dadurch, daß die Matrix komprimierbar und porös ist, kann unter Bedingungen, bei denen das biologische Material von der Oberfläche der Matrix in eine Elutionsflüssigkeit freigesetzt wird, durch Kompression der Matrix eine Gewinnung einer Lösung des isolierten biologischen Materials gewonnenen werden. Unter Kompression wird ein Zusammendrücken des räumlich ausgedehnten Gebildes verstanden, wobei Flüssigkeit aus dem Innenvolumen des Gebildes herausgedrückt wird. Dadurch erhöht sich der Anteil des unlöslichen Materials an dem Gesamtgebilde, während sich der Anteil des verbleibenden Innenvolumens reduziert. Im Sinne der Erfindung wird die Matrix um bevorzugt mehr als 40 % und besonders bevorzugt mehr als 60 % komprimiert. Das Ausmaß der Kompression hängt u. a. auch von der gewünschten Menge an Flüssigkeit ab, die das biologische Material enthalten soll. Die aus der Matrix austretende Flüssigkeit kann in jeder gewünschten Weise gewonnen und weiterverarbeitet werden.

Als Elutionsflüssigkeit wird im Sinne der Erfindung eine Flüssigkeit verstanden, in der das zu isolierende biologische Material gelöst werden kann. Sie hat eine derartige chemische Zusammensetzung, daß das Gleichgewicht zwischen an das unlösliche Material und der Lösung auf Seite der Lösung verschoben ist. Für den Fall der Isolierung von Nukleinsäuren wird daher als Eulutionsflüssigkeit eine Lösung verwendet, die einen geringeren Salzgehalt hat als die Lösung, aus der die Nukleinsäuren an das unlösliche Material gebunden wurde.

Das erfindungsgemäße Verfahren findet bevorzugt in einem System statt. Hierzu wird die komprimierbare poröse Matrix mit dem daran gebundenen biologischen Material in ein Elutionsgefäß eingebracht. Bevorzugt ist die komprimierbare poröse Matrix Teil eines Gefäßes, welches in der Form an den Innenraum des Elutionsgefäßes angepaßt ist. Zur Freisetzung des biologischen Materials wird die Matrix mit Elutionsflüssigkeit versetzt und anschließend mit Hilfe eines Stempels komprimiert, wobei eine Möglichkeit zum Austritt der Flüssigkeit entweder im Stempel oder dem die Matrix enthaltenden Gefäß oder dem Elutionsgefäß oder zwischen diesen Bauteilen vorgesehen wird.

Die fiir die Konstruktion der genannten Gefäße verwendeten Materialien sind bevorzugt solche, die in Spritzgußverfahren in die gewünschte Form zu bringen sind, z. B. Kunststoffe wie Polystyrol, besonders bevorzugt, Polypropylen, ggf. mit geeigneten Zusätzen.

In Fig. 1 ist ein Formkörper C in Form eines Gefäßes gezeigt, welches in seinem unteren Teil eine komprimierbare poröse Matrix enthält (Längsschnitt).

In Fig. 2 ist ein erfindungsgemäßes System in einem Zustand gezeigt, bei dem die Matrix komprimiert ist (Längsschnitt).

In Fig. 3 ist ein erfindungsgemäßer Stempel zu Kompression der Matrix und zur Aufnahme der Elutionsflüssigkeit gezeigt (Längsschnitt).

In Fig. 4 ist ein Elutionsgefäß D gezeigt, welches in vorteilhafterweise zur Auspressung einer komprimierbaren porösen Matrix geeignet ist (Längsschnitt).

In Fig. 5 sind schematisch die Verfahrensschritte und beteiligten Konstrukte für ein Verfahren zur Isolierung eines biologischen Materials gemäß der Erfindung gezeigt.

Im Folgenden werden die in den Figuren beschriebenen Gegenstände näher erläutert:

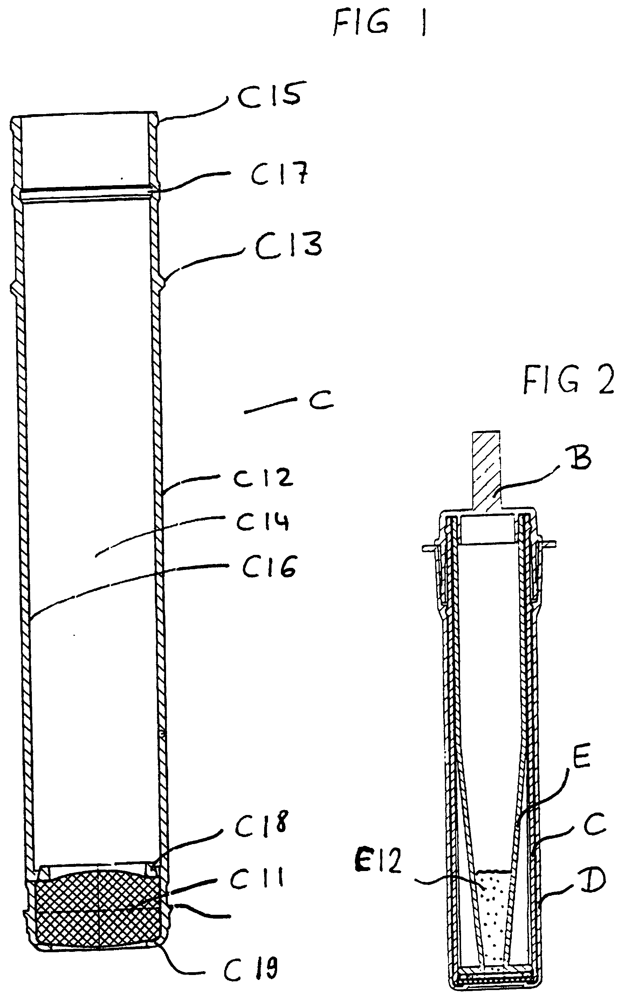

In Fig. 1 ist im Längsschnitt ein Formkörper (C) abgebildet, welcher eine im wesentlichen eine hohlzylinderische Gestalt aufweist und der eine obere und eine untere Öffnung besitzt. Im unteren Teil ist durch Verengungen im Querschnitt des Röhrchens die komprimierbare poröse Matrix (C11) fixiert. Diese Matrix wird nach unten hin durch einen Rand (C19) und nach oben hin durch einen umlaufenden, abbrechbaren Steg (C18) gehalten. Durch Vorsehen einer Sollbruchstelle entlang der Innenwand (C16) des Formkörpers kann dieser Steg abgebrochen und in Richtung auf die Matrix (C11) geschoben werden. Der Formkörper (C) enthält außerdem eine äußere Kontur (C12) und einen Innenraum, der als Hohlkörper ausgebildet ist (C14). Des weiteren befinden sich vorzugsweise an diesem Formkörper Mittel zur Fixierung (C17) eines Stempels (E) im Formkörper. Bevorzugt ist dieses Mittel so ausgebildet, daß der Stempel in einer Position fixiert wird, in der er die Matrix (C11) komprimiert. Darüber hinaus weist der Formkörper (C) bevorzugt Mittel (C15) zur Fixierung eines Deckels auf der oberen Öffnung auf. Ferner können Mittel (C13) zur Fixierung des Formkörpers im Elutionsgefäß (D) vorgesehen sein.

In Fig. 2 ist ein besonders bevorzugtes System gemäß der Erfindung gezeigt. Es besteht aus den Komponenten Elutionsgefäß (D), Formkörper (C), Stempel (E) und Deckel (B). Der Deckel (B) ist hierbei so gestaltet, daß er die Gesamtheit der Gefäße verschließt. In Fig. 2 ist das System in einer Form gezeigt, in der die ursprünglich in der Matrix befindliche Elutionsflüssigkeit aus dieser Matrix heraus in den Innenraum (C12) des Stempels gedrückt wurde.

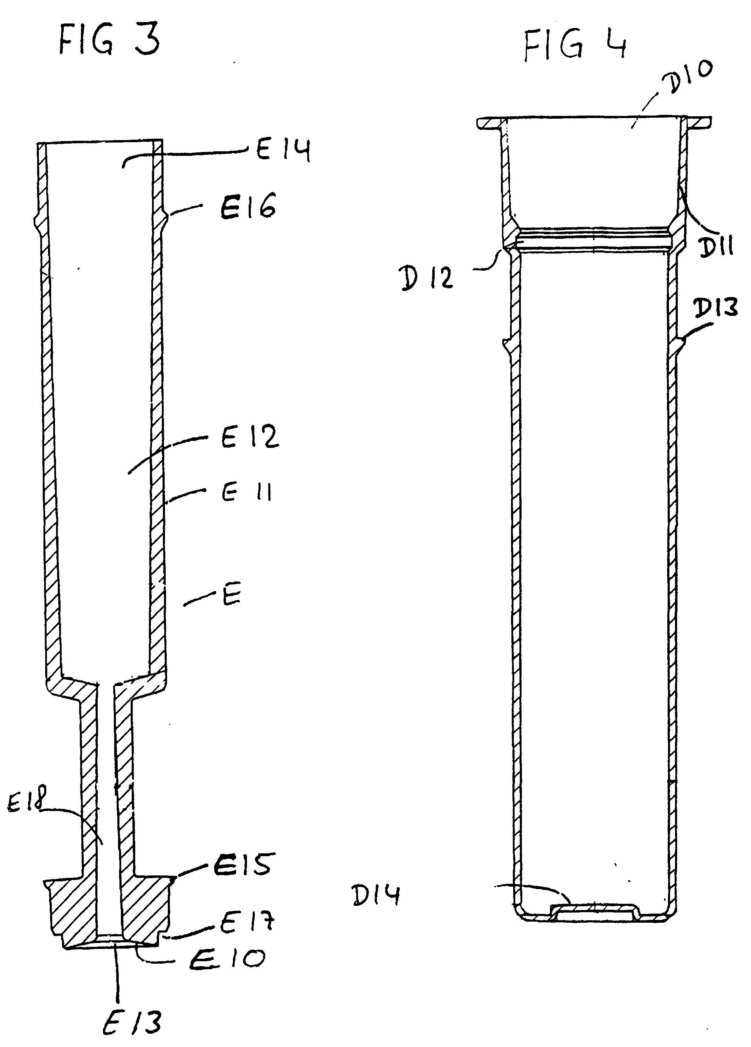

In Fig. 3 ist ein besonders bevorzugter Stempel (E) zum Auspressen der komprimierbaren porösen Matrix gezeigt. Er ist gekennzeichnet durch einen Dichtring (E15) im unteren Bereich, der den Zwischenraum zwischen Formkörper (C) und Elutionsgefäß (D) abdichtet und somit verhindert, daß ausgepreßte Lösung in den entstehenden Kapillarspalt gelangen kann. Weiter besitzt der Stempel (E) im oberen Bereich einen Einrastring (E16), der beim Einschieben in die Einrastkerbe (C17) des Formkörpers (C) irreversibel den Stempel im Formkörper fixiert. Durch den fixierten Formstempel (D) wird ein ausreichender Druck auf die Matrix ausgeübt, um Flüssigkeit aus der Matrix herauszupressen.

Über die Einrastlänge des Stempels (E) und die Lage der Einrastkerbe im Formkörper (C) kann der Auspreßdruck auf die Matrix in einem bestimmten Kraftbereich festgelegt werden. Die untere Geometrie des Stempels (E) stellt einen Auspreßstempel dar, dessen formschlüssige Geometrie der Andrucksfläche (E10) geeignet ist, das Auspressen der Matrix zu gewährleisten. Die Form des Stempels ist so gestaltet, daß eine Minimierung des Totvolumens erreicht wird. Die Aussparungen (E17) gewährleisten die Aufnahme des abgebrochenen Stegs (C18).

Zur Gewinnung und Aufbewahrung der aus der Matrix ausgepreßten Lösung besitzt der Unterbereich des Stempels eine traversal durch das gesamte Modul geführte zylindrische Bohrung mit einem relativ kleinen Durchmesser und einer geeigneten Höhe, die sowohl für die Aufnahme der ausgepreßten, nukleinsäurehaltigen Lösung, als auch für die Entnahme mittels einer Pipettenspitze geeignet ist. In Fig. 3 ist der Fall gezeigt, daß die Bohrung (E18) relativ eng ist, im Vergleich zu dem möglichen Innenvolumen des Stempels. Die Bohrung (E18) endet einerseits in der Öffnung (E13) in der Andrucksfläche. Andererseits führt sie in den Innenraum des Stempels (E12).

Der obere Bereich des Stempels stellt ebenfalls eine traversal verlaufende Bohrung dar, welche als Auffanggefäß fiir die gewonnene Lösung des biologischen Materials dient und im Vergleich zum unteren Bereich einen relativ großen Durchmesser und eine geeignete Höhe besitzt. Der Durchmesser und die Höhe der Bohrung so gewählt, daß sie für das Einführen einer Pipette in den Innenraum (E12) und die Entnahme der über die Bohrung (E18) in den Innenraum eingetretenen Lösung geeignet ist.

In Fig. 4 ist im Längsschnitt ein bevorzugtes Elutionsgefäß (D) gezeigt. Es weist eine Einlaßöffnung (D10) auf, durch welche Flüssigkeiten bzw. weitere Funktionselemente in das Elutionsgefäß eingeführt werden können. Außerdem weist es einen inneren Rand (D11) auf, der zur Befestigung eines Deckels (B) in der Lage ist. Ebenfalls im oberen Teil des Elutionsgefäßes befindet sich eine Einrastkerbe (D12) auf, mit der der Formkörper (C) über dessen Mittel (C13) fixiert werden kann. Ein weiteres bevorzugtes Merkmal sind Mittel zur Befestigung des Elutionsgefäßes (D13) in einer Lochplatte geeigneten Durchmessers. Ein besonders vorteilhaftes Merkmal ist auch die Andrucksfläche (D14) im unteren Teil des Elutionsgefäßes. Durch Anpassung dieser Fläche an den unteren Teil des Formkörpers kann das Totvolumen innerhalb des Gefäßes reduziert werden. Dies führt zur besonders effektiven Gewinnung des biologischen Materials.

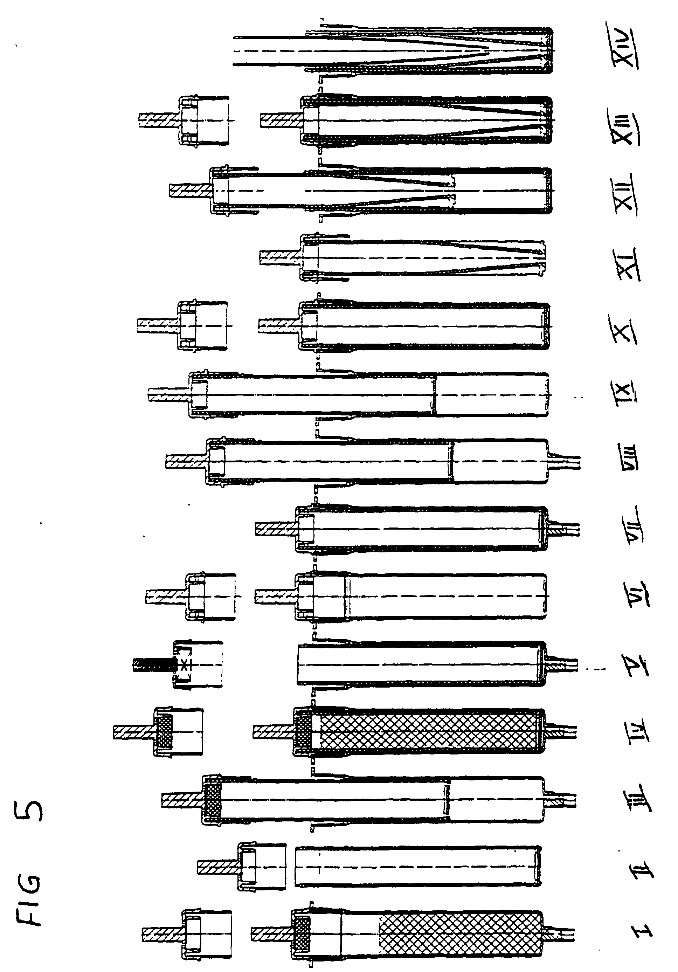

In einer besonderen Ausführungsform fiir die Aufarbeitung nukleinsäurehaltiger Probenlösungen, werden folgende Arbeitsschritte durchgeführt (siehe Fig. 5). In einem ersten Schritt (I) wird eine zellhaltige Probenflüssigkeit in einem Probegefäß (A) mit einem Material inkubiert, an welches die Zellen gebunden werden, aus denen Nukleinsäuren gewonnen werden sollen. Hierzu kann dieses Material entweder spezifische Bindeeigenschaften für die Oberfläche der Zellen aufweisen, z. B. durch Immobilisierung von Antikörpern gegen Oberflächenantigene oder ein Absorbermaterial (A16), es kann jedoch auch ein Material mit Filtereigenschaften (A15) vorgesehen sein, durch welches die Zellen zurückgehalten werden, wenn die Flüssigkeit durch das Material durchtritt, z. B. aus dem Probengefäß entfernt wird. Bedingungen für die Immobilisierung von Zellen an Oberflächen sind dem Fachmann bekannt, z. B. aus Methods in Enzymology Vol. 171, Biomembranes / Part R Transport Theory: Cell and Model Membranes, Edited by Sidney Fleischer, Becca Fleischer, Department of Molecular Biology, Vanderbilt University, Nashville, Tennessee.

Während der Inkubation ist das Probengefäß bevorzugt durch einen Deckel (B) verschlossen, um aktiven bzw. passiven Kontaminationsschutz zu gewährleisten.

In einem weiteren Schritt wird die Flüssigkeit aus dem Probengefäß entfernt, während Zellen, deren Nukleinsäuren isoliert werden sollen, in an das Material gebundenem Zustand im Probengefäß zurückbleiben. Sofern es sich bei dem zellbindenden Material um partikuläre Materialien handelt, kann ein Zurückhalten auch dadurch erreicht werden, daß das Material magnetisch ist und ein Magnetfeld von außen an das Probengefäß angelegt wird, welches so stark ist, daß das partikuläre Material im Probengefäß zurückbleibt, wenn die Flüssigkeit entfernt wird. Das Entfernen der Flüssigkeit kann auf verschiedenste Weise geschehen. Beispielsweise kann die Flüssigkeit durch eine räumlich von der Einlaßöffnung (A10) getrennte Auslaßöffnung (A11) entfernt werden. Sofern die Auslaßöffnung im unteren Teil des Probengefäßes und unterhalb der zurückgehaltenen Zellen gelegen ist, kann die Flüssigkeit, z. B. unter Anlegen eines leichten Vakuums, abgesaugt werden. Hierzu kann beispielsweise an der Auslaßöffnung ein Ventil vorgesehen sein, welches sich durch Anlegen von Unterdruck öffnet.

Zur weitgehenden Entfernung eventuell störender Probenbestandteile von den Zellen können ein oder mehrere Waschschritte vorgesehen werden. Hierzu wird in das Probengefäß eine Waschflüssigkeit eingefüllt, in der sich eventuelle Verunreinigungen lösen, die jedoch die Bindung der Zellen an die Oberfläche des zellbindenden Materials nicht wesentlich beeinträchtigen. Solche Waschlösungen sind dem Fachmann z. B. aus den Zellseparationsprotokollen bzw. aus entsprechenden Reinigungskitsprotokollen für Nukleinsäuren bekannt. Sie richten sich im wesentlichen nach der Art der Bindung der Zellen an das Material.

Nachdem gegebenenfalls die letzte Waschlösung aus dem Probengefäß (A) entfernt wurde, werden die gereinigten, angereicherten Zellen mit einer geeigneten Lyseflüssigkeit zur Freisetzung der Nukleinsäuren aus den Zellen in Kontakt gebracht. Die Reagenzien dieser Lyselösung richten sich weitgehend nach der Art der immobilisierten Zellen. Sofern es sich bei den Zellen um Bakterien handelt, enthält die Lyselösung bevorzugt Proteinase K zum Abbau der Zellwand. Gewünschtenfalls wird die Lyse durch Erhitzen bzw. Abkühlen sowie Mischen der Reaktionsmischung unterstützt. Sofern es sich bei dem zellbindenden Material um magnetische Partikel handelt, kann die Mischung auch mittels Magneten vorgenommen werden. Außerdem ist eine Mischung durch Schütteln des Probengefäßes möglich. Am Ende dieses Aufschlusses liegen die zu isolierenden Nukleinsäuren frei in der Lösung vor.

Auch während der Lyse ist das Reaktionsgefäß bevorzugt durch einen Deckel verschlossen, um Kontaminationen aus der Umgebung zu verhindern. Nach Ende der Lyse wird der Deckel, bevorzugt mit Hilfe einer entsprechenden mechanischen Vorrichtung, entfernt. Danach wird in das Probengefäß, welches eine Mischung von Abbauprodukten der Zellen sowie die Nukleinsäuren enthält, ein Formkörper (C) eingeführt, dessen äußere Kontur (C12) auf die innere Kontur (A10) des Probengefäßes abgestimmt ist. Dieser Formkörper ist hohl und in Richtung auf das Probengefäß und die Reaktionsmischung hin durch einen Filter (C11) verschlossen. Die Einführung des Formkörpers (C) erfolgt bevorzugt mit Hilfe eines Bauelementes (B20) des Deckels (B), der außerdem ein Bauelement (B10) enthält, welches zum Verschluß des Probengefäßes geeignet ist. In diesem Fall wird der Formkörper gleichzeitig mit dem Verschluß des Probengefäßes in das Probengefäß eingeführt (II, III). Während dieses Vorgangs wird außerdem die Reaktionsmischung durch den Filter (C11) in den Hohlraum (C14) des Formkörpers eindringen (IV). Durch das Vorsehen des Filters können einerseits große Partikel an dem Eintritt in den Hohlraum gehindert werden und andererseits kann, wenn der Filter nukleinsäurebindende Eigenschaften hat, schon während des Durchtritts der Reaktionsmischung eine Bindung der Nukleinsäuren an den Filter erreicht werden. In diesem Fall ist es zweckmäßig, ein glasfaserhaltiges Filtermaterial zu wählen.

In einem nächsten Schritt wird die verbleibende Lysereaktionsmischung aus der durch A und C gebildeten Vorrichtung entfernt, z. B. durch Absaugen durch eine untenliegende Auslaßöffnung (A12) im Probengefäß. Auch die in den Hohlkörper (C14) des Formkörpers eingedrungene Lösung wird somit entfernt, so daß der Filter möglichst keine Flüssigkeitsreste mehr enthält (V).

Gleichzeitig oder anschließend wird ein Elutionsgefäß (D) zur Aufnahme des Formkörpers (C) vorbereitet. Ein gegebenenfalls auf diesem Gefäß befindlicher Deckel (B) wird entfernt VI). Bevorzugt wird vor Überführung des Formkörpers (C) in das Elutionsgefäß (D) eine Elutionslösung in das Elutionsgefäß vorgelegt, z. B. einpipettiert. Die Zusammensetzung der Elutionslösung richtet sich nach der Art der Bindung der Nukleinsäuren an das Material in der Matrix (C11). Sie enthält Reagenzien, unter deren Einwirkung die immobilisierten Nukleinsäuren von dem Material eluiert, d. h. gelöst, werden. Ein neuer Deckel (B) wird auf das Probengefäß (A) und den Formkörper (C) aufgesteckt (VII).

Zur Entnahme des Formkörpers (C) aus dem Probengefäß (A) wird zunächst der Deckel (B) entfernt (VIII). Die Kombination aus Deckel und Formkörper wird anschließend in das Elutionsgefäß eingeführt. Bevorzugt enthält der Formkörper (C) Mittel (C13) zur Fixierung des Formkörpers im Elutionsgefäß (D), die bewirken, daß der Formkörper nur unter Zerstörung des Formkörpers (C) oder des Gefäßes (D) oder mit einer Kraft, die größer ist als die Kraft die zur Lösung des Deckels (B) vom Formkörper (C) erforderlich ist, aus dem Gefäß (D) entfernt werden kann. Eine Entfernung des Formkörpers aus dem Elutionsgefäß ist nicht beabsichtigt.

Während des Eindringens des Formkörpers (C) in das Elutionsgefäß dringt vorgelegte Elutionsflüssigkeit in die Matrix (C11) ein und die löst die immobilisierte Nukleinsäure von dem unlöslichen Material ab. Je nach Menge der vorgelegten Elutionslösung wird entweder nur die Matrix mit der Elutionslösung getränkt oder dringt die Elutionslösung mit den wieder gelösten Nukleinsäuren in den Hohlkörper (C14) ein. Damit die Elution der Nukleinsäuren möglichst vollständig verläuft, sollte die Innenkontur des Elutionsgefäßes möglichst dicht an die Außenkontur des Formkörpers angepaßt sein.

In einem folgenden Schritt wird der Deckel (B) von Formkörper (C) und Elutionsgefäß (D) entfernt (X), um einen Stempel (E) aufzunehmen (XI) und in den Hohlraum des Formkörpers (C) einzuführen (XII). Der Stempel wird so kräftig gegen die Matrix (C11) gepreßt, daß Flüssigkeit aus dem Filter durch eine in der Andrucksfläche befindliche Öffnung in einen Innenraum des Stempels stattfindet. Dieser Vorgang ist besonders effektiv, wenn die Andrucksfläche in ihrer äußeren Kontur zumindest in dem Bereich, in dem die Auspressung stattfinden soll, an die innere Kontur des Formkörpers (C) angepaßt ist. Der Stempel (E) kann bevorzugt in dieser Lage, z. B. durch einen Einrastring, fixiert werden. Da die so gebildete Vorrichtung durch den Deckel relativ gut verschlossen ist, kann die nukleinsäurehaltige Lösung in der Vorrichtung aufbewahrt werden.

Zur Entnahme einer gewünschten Menge an Nukleinsäurelösung kann der Deckel entfernt (XIII) und über eine Öffnung des Innenraums des Stempels die gewünschte Menge entnommen werden, z. B. in einem Pipettiervorgang (XIV). Anschließend kann der Deckel wieder aufgesetzt werden.

Bevorzugt weist das erfindungsgemäße Verfahren folgende Schritte auf

a) Bindung des biologischen Materials an eine komprimierbare poröse Matrix (C11),

b) Zugabe einer Elutionsflüssigkeit in ein Elutionsgefäß (D),

c) Einführen der komprimierbaren porösen Matrix (C11), besonders bevorzugt in einem Formkörper (C),

d) Einführen eines Stempels (D) in den Formkörper (C) und das Elutionsgefäß (D), wobei der Stempel eine Andrucksfläche besitzt, die zur Kompression der Matrix geeignet ist und wobei der Stempel einen Innenraum (E12) zur Aufnahme der aus der Matrix herausgepreßten Flüssigkeit aufweist,

e) Einrasten des Stempels im Formkörper, so daß die Matrix sich im komprimierten Zustand befindet.

Hierbei können die Schritte b und c in der Reihenfolge ausgetauscht sein. Die in den Innenraum (C12) eingedrungene Elutionsflüssigkeit mit dem biologischen Material kann anschließend in dem System aufbewahrt werden (vorzugsweise bei geschlossenem Deckel) oder mit Hilfe einer Pipette entnommen und auf beliebige Weise weiterbearbeitet werden.

Das erfindungsgemäße Verfahren eignet sich im besonderen Maße zur Erzeugung relativ hoch konzentrierter Lösungen von biologischen Materialien. Gemäß der Erfindung wird eine besonders aerosolbildungsarme Gewinnung von Nukleinsäuren aus der Matrix erreicht. Außerdem erleichtert die Art der Bearbeitungsschritte stark die Automation der Arbeitsabläufe und Verfahren zur Isolierung biologischer Materialien.

Ebenfalls Gegenstand der Erfindung ist ein System zur Isolierung eines biologischen Materials, enthaltend die Komponenten:

- ein Elutionsgefäß (D);

- eine komprimierbare poröse Matrix und

- einen Stempel (E), mit welchem die Matrix komprimiert werden kann.

Weitere bevorzugte Merkmale sind im erfindungsgemäßen Verfahren geschildert.

Die Erfindung wird durch das folgende Beispiel näher beschrieben.

**Beispiel 1:**

Eine bevorzugte Ausführungsform des Stempels stellt ein hohlzylinderförmiges Kunststoffröhrchen (E) dar, welches unterschiedliche Durchmesser (D [unten] = 1 mm, D [oben] = 5 mm) im Innenraum besitzt.

Der Stempel kann durch folgende Abmaße beschrieben werden:

| | |
|---|---|
| Länge | 38,6 mm |
| Außendurchmesser | 5,8 mm |
| Innendurchmesser | 5,0 mm. |

Der untere Teil besitzt eine Stempelgeometrie mit zentrischer Bohrung (E13), der obere Teil stellt ein nach oben offenes Auffanggefäß (E12) dar.

Die äußere Mantelfläche (E) besitzt im oberen Bereich Einrastlippen (E16), welche zur Verankerung und Fixierung im Formkörper (C) geeignet sind. Die umlaufende Einrastlippe befindet sich 3,5 mm unterhalb der Oberkante, sie besitzt eine Tiefe von 0,3 mm und eine Höhe 0,25 mm. Im unteren Bereich besitzt der Stempel Dichtungslippen (E15), welche den Stempel gegenüber dem Formkörper abdichten. Sie sind 0,28 mm erhaben und 0,10 mm breit.

Die obere Öffnung des Stempels besitzt eine innere Geometrie zur Kopplung eines multifunktionellen Deckels (B) und des Elutionsgefäßes (D). Der Stempel (E) ist so gestaltet, daß er in den Formkörper (C) eingeführt werden kann und zusammen mit dem Deckel (B) das gesamte System verschließt.

**Beispiel 2:**

Biologische Materialien / Chemikalien / Devices

| | |
|---|---|
| Probenmaterial | Längenstandard II, Fa. Boehringer Mannheim |
| Bindungspuffer | Qiagen AL 1 / AL 2 (4 Teile / 1 Teil), Fa. Qiagen |
| Waschpuffer | Qiagen AW-Puffer, Fa. Qiagen |
| Elutionspuffer | 10 mM Tris-Puffer pH 9,0 |

Probengefäß (A)
Probengefäß-Deckel (B)
Formkörper (C) mit einer porösen Matrix aus Glasvlies (C11)
Elutionsgefäß (D)
Elutionsgefäß-Deckel (E)
Auspreßstempel-Formkörper (F)

Ansatz der Probenlösungen

| | | |
|---|---|---|
| Probe | 200 µl | (6 µl Längenstandard II in PBS-Puffer gelöst) |
| Proteinase K | 25 µl | (AL 1 und AL 2-Puffer in Verhältnis 4+1 gemischt) |
| Bindungspuffer | 200 µl | |
| Ethanol | 210 µl | |
| Gesamtvolulmen pro Ansatz | 635 µl | |

Durchführung des Beispieles

Probengefäß (A) wird mit 200 µl Probe, 25 µl Proteinase K-Lösung und 200 µl Bindungspuffer gefüllt. Das Probengefäß (A) wird mit dem Probengefäß-Deckel (B) verschlossen. Die Flüssigkeiten werden im geschlossenen Gefäß gemischt. Danach erfolgt die Inkubation bei 70°C/10 min (Aufschluß der Zellen) mit anschließender Abkühlungsphase auf 20°C in 3 min. Das Probengefäß (A) wird geöffnet und es erfolgt eine Zugabe von 210 µl Ethanol. Das Probengefäß (A) wird verschlossen und die Lösung gemischt.

Das Probengefäß (A) wird geöffnet. Der Probengefäß-Deckel (B) holt den Formkörper (C) mit einer porösen Matrix aus der Vorlage. Der Formkörper mit der Glasvlies-Matrix wird durch die Eingangsöffnung in das mit Flüssigkeit gefüllte Probengefäß eingebracht. Die im Probengefäß (A) vorhandene Flüssigkeit durchströmt von unten das Glasvlies während des Einführungsvorganges. Die frei bewegliche Nukleinsäure bindet an der Glasvlies-Matrix. Im nächsten Schritt wird die im Formkörper (C) stehende Flüsisgkeit nach unten abgesaugt. Dabei strömt die Flüssigkeit nochmals durch das Glasvlies und noch nicht gebundene Nukleinsäure wird an die Matrix fixiert.

Die Glasvlies-Matrix wird 2mal mit 500 µl Waschpuffer gereinigt. Die über der Matrix stehende Pufferlösung wird durch das Glasvlies abgesaugt. Anschließend wird die Matrix mit der gereinigten Nukleinsäure bei 50°C/3 min getrocknet. Der Probengefäß-Deckel (B) wird verworfen.

Der Formkörper (C) mit der Nukleinsäure wird mittels Elutionsgefäß-Deckel (E) von dem Probengefäß (D) in einen vorbereiteten Elutionspuffer mit 200 µl Elutionspuffer überführt. Der Elutionspuffer löst die Nukleinsäure von der Glasvlies-Matrix ab. Die Nukleinsäure-Lösung steht teilweise über der Matrix.

Die Matrix im Formkörper wird in einem weiteren Schritt durch das Einführen des Auspreßstempel-Formkörper (F) mit dem Elutionsgefäß-Deckel (E) komprimiert, um das Totvolumen der Nukleinsäure-Lösung zu minimieren.

Nukleinsäure-Ausbeuten

Die Versuche wurden mit zwei verschiedenen Nukleinsäureprobe-Konzentrationen durchgeführt. Folgende Ergebnisse konnten ermittelt werden:

Versuchsreihe 1: Probenkonzentration: 6 µg Nukleinsäure/200µl Elutionslösung

| Versuchs-Nummer | isolierte gebundene Nukleinsäure (µg) | Ausbeute (%) |
|---|---|---|
| Versuch 1 | 1,4 µg | 22,9 |
| Versuch 2 | 1,6 µg | 27,5 |
| Versuch 3 | 1,2 µg | 23,8 |
| Versuch 4 | 1,5 µg | 29,8 |
| Versuch 5 | 1,6 µg | 35,2 |

Versuchsreihe 2: Probenkonzentration: 20 µg Nukleinsäure/200 µl Elutionslösung

| Versuchs-Nummer | isolierte gebundene Nukleinsäure (µg) | Ausbeute (%) |
|---|---|---|
| Versuch 1 | 3,7 µg | 28,0 |
| Versuch 2 | 6,4µg | 38,8 |
| Versuch 3 | 4,8 µg | 40,0 |
| Versuch 4 | 4,1 µg | 30,3 |
| Versuch 5 | 5,1 µg | 37,8 |

**Bezugszeichenliste**

**A    Probengefäß**

10    Einlaßöffnung
11    Auslaßöffnung
12    elastisches Material
13    Seitenwand der Austrittsstutzen A14
14    Austrittsstutzen
15    Filtermaterial
16    Absorbermaterial
17    innere Form
18    elastischer Schlauch
19    Außenform
20    umlaufender Steg
21    Halteelement
22    Element zur Fixierung von weiteren Funktionselementen
23    Spiralfeder
24    Kugel
25    Nadel
26    Blattfeder

**B    Deckel**

10    Bauelement zum Verschließen des Probengefäßes A
11    Bauelement zum Ergreifen des Formkörpers C
12    Bauteil zum Ergreifen des Deckels
13    Luftdurchtrittsöffnung
14    Filterelement
15    Element zum Einrasten von B10 in A
16    Dichtlippe

17    Teil des Deckels, der die Öffnung überdeckt

**C    Formkörper**

11    poröse Matrix
12    äußere Kontur
13    Mittel zur Fixierung des Formkörpers im Elutionsgefäß
14    Hohlkörper
15    Mittel zur Befestigung eines Deckels
16    innere Kontur
17    Mittel zur Fixierung eines Stempels
18    umlaufender Steg
19    Rand

**D    Elutionsgefäß**

10    Einlaßöffnung
11    Deckelbefestigungsrand
12    Einrastkerbe
13    Mittel zur Fixierung in einer Lochplatte
14    Andrucksfläche

**E    Stempel**

10    Andrucksfläche
11    Außenkontur
12    Innenraum
13    Öffnungen in der Andrucksfläche
14    Entnahmeöffnung
15    Dichtung
16    Einrastring
17    Aussparung

**Patentansprüche**

1.  Verfahren zur Isolierung von Proteinen oder Nukleinsäuren durch

    -    Bereitstellung von an eine komprimierbare poröse Matrix (C11) gebundenen Proteinen oder Nukleinsäuren,

    -    Kompression der Matrix unter Bedingungen, bei denen die Proteine oder Nukleinsäuren von der Oberfläche der Matrix in eine Elutionsflüssigkeit freigesetzt werden,

    und

    -    Durchtritt der Elutionsflüssigkeit durch eine oder mehrere Öffnungen (E13) der Andrucksfläche (E10) eines Stempels in einen Innenraum des Stempels bei Kompression der Matrix mit Hilfe des Stempels,

    wobei

    -    Die Matrix (C11) im unteren Teil eines Formkörpers (C) fixiert ist, der sich in einem Elutionsgefäß (D) befindet,

    -    die Außenkontur (E11) des Stempels (E) flüssigkeitsdicht gegen die Kontur (C12) des Formkörpers (C) abschließt, wenn der Stempel gegen die Matrix (C11) drückt, und

    -    die Matrix von der Andrucksfläche (E10) des Stempels gegen einen flüssigkeitsdichten Teil des Elutionsgefäßes (D) gedrückt wird.

**2.** Verfahren gemäß Anspruch 1, **dadurch gekennzeichnet, daß** Nukleinsäuren isoliert werden.

**3.** System zur Isolierung von Proteinen oder Nukleinsäuren, enthaltend die Komponenten:

- ein Elutionsgefäß (D);
- eine komprimierbare poröse Matrix (C11), die im unteren Teil eines Formkörper (C) fixiert ist, der sich in einem Elutionsgefäß (D) befindet und
- einen Stempel (E), mit welchem die Matrix (C11) komprimiert werden kann

**dadurch gekennzeichnet, daß**

- die Außenkontur (E11) des Stempels (E) derart gestaltet ist, daß diese flüssigkeitsdicht gegen die Kontur (C12) des Formkörpers (C) abschließt, wenn der Stempel gegen die Matrix (C11) drückt,

- der Stempel in der Andrucksfläche eine oder mehrere Öffnungen (E13) zum Durchtritt der Elutionsflüssigkeit in einen Innenraum des Stempels aufweist, und

- der Teil des Elutionsgefäßes, gegen den die Andrucksfläche (E10) des Stempels bei Kompression der Matrix drückt, flüssigkeitsdicht ist.

**4.** System gemäß Anspruch 3, **dadurch gekennzeichnet, daß** die Matrix von der Andrucksfläche (E10) des Stempels (E) gegen einen an die Form der Andrucksfläche angepaßten Teil (D14) des Elutionsgefäßes (D) gedrückt wird.

**5.** System gemäß Anspruch 3, **dadurch gekennzeichnet, daß** der Innenraum (E12) des Stempels im wesentlichen konisch auf eine Öffnung (E13) in der Andrucksfläche des Stempels zuläuft.

**6.** System gemäß Anspruch 3, **dadurch gekennzeichnet, daß** die Matrix in einem Elutionsgefäß (D) durch einen Stempel (E) komprimiert wird und der Stempel direkt oder indirekt in den Formkörper (C) einrasten kann, wenn sich die Matrix in einem komprimierten Zustand befindet.

**Claims**

**1.** Method for isolating proteins for nucleic acids by

- preparing proteins or nucleic acids which are bound to a compressible porous matrix (C11),

- compressing the matrix under conditions where the proteins or nucleic acids are released from the surface of the matrix into an elution liquid,

and

- passing the elution liquid through one or several openings (E13) of the pressure face (E10) of a plunger into an interior space of the plunger when the matrix is compressed with the aid of the plunger,

wherein

- the matrix (C11) is attached in the lower part of a shaped body (C) which is located in an elution vessel (D)

- the outer contour (E11) of the plunger (E) makes a liquid-tight seal against the contour (C12) of the shaped body (C) when the plunger presses against the matrix (C11) and

- the pressure face (E10) of the plunger presses the matrix against a liquid-tight part of the elution vessel (D).

**2.** Method as claimed in claim 1, **characterized in that** nucleic acids are isolated.

**3.** System for isolating proteins or nucleic acids containing the components:

- an elution vessel (D);
- a compressible porous matrix (Cl 1) which is attached in the lower part of a shaped body (C) which is located in an elution vessel (D) and
- a plunger (E) which can compress the matrix (C11)

**characterized in that**

- the outer contour (E11) of the plunger (E) is designed such that it makes a liquid-tight seal against the contour (C12) of the shaped body (C) when the plunger presses against the matrix (C11),

- the pressure face of the plunger has one or several openings (E13) to allow passage of the elution liquid into an interior space of the plunger and

- the part of the elution vessel which presses against the pressure face (E10) of the plunger when the matrix is compressed is liquid-tight.

4. System as claimed in claim 3, **characterized in that** the pressure face (E 10) of the plunger (E) presses the matrix against a part (D14) of the elution vessel (D) which matches the shape of the pressure face.

5. System as claimed in claim 3, **characterized in that** the interior space (E12) of the plunger is essentially conically tapered towards an opening (E13) in the pressure face of the plunger.

6. System as claimed in claim 3, **characterized in that** the matrix is compressed in an elution vessel (D) by a plunger (E) and the plunger can engage directly or indirectly in the shaped body (C) when the matrix is in a compressed state.


**Revendications**

1. Procédé pour isoler des protéines ou des acides nucléiques, consistant à

- procurer des protéines ou des acides nucléiques liés à une matrice poreuse (C11) apte à être comprimée,

- comprimer la matrice dans des conditions dans lesquelles les protéines ou les acides nucléiques sont libérés de la surface de la matrice dans un liquide d'élution

et

- faire passer le liquide d'élution à travers une ou plusieurs ouvertures (E13) de la surface de pression (E10) d'un poinçon dans un espace interne du poinçon lors de la compression de la matrice à l'aide du poinçon,

dans lequel

- la matrice (C11) est fixée dans la partie inférieure d'un corps moulé (C) qui se trouve dans un récipient d'élution (D),

- le contour externe (E11) du poinçon (E) épouse le contour (C12) du corps moulé (C) d'une manière étanche aux liquides, lorsque le poinçon est comprimé contre la matrice (C11), et

- la matrice est pressée par la surface de pression (E10) du poinçon contre une partie du récipient d'élution (D) étanche aux liquides.

2. Procédé selon la revendication 1, **caractérisé en ce qu'**on isole des acides nucléiques.

3. Système pour isoler des protéines ou des acides nucléiques, contenant les composants ci-après :

- un récipient d'élution (D) ;
- une matrice poreuse (C11) apte à être comprimée, qui est fixée dans la partie inférieure d'un corps moulé (C) qui se trouve dans un récipient d'élution (D) et

- un poinçon (E) à l'aide duquel la matrice (C11) peut être comprimée

**caractérisé en ce que**

- le contour externe (E11) du poinçon (E) est réalisé de telle sorte qu'il épouse le contour (C12) du corps moulé (C) d'une manière étanche aux liquides, lorsque le poinçon est comprimé contre la matrice (C11),

- le poinçon présente, dans sa surface de pression, une ou plusieurs ouvertures (E13) pour le passage du liquide d'élution dans un espace interne du poinçon, et

- la partie du récipient d'élution contre laquelle est comprimée la surface de pression (E10) du poinçon lors de la compression de la matrice est étanche aux liquides.

4. Système selon la revendication 3, **caractérisé en ce que** la matrice est comprimée par la surface de pression (E10) du poinçon (E) contre une partie (D14) du récipient d'élution (D) dont la forme épouse celle de la surface de pression.

5. Système selon la revendication 3, **caractérisé en ce que** l'espace interne (E12) du poinçon s'étend sous une forme essentiellement conique jusqu'à une ouverture (E13) pratiquée dans la surface de pression du poinçon.

6. Système selon la revendication 3, **caractérisé en ce que** la matrice est comprimée dans un récipient d'élution (D) par un poinçon (E) et le poinçon peut venir s'encliqueter de manière directe ou indirecte dans le corps moulé (C) lorsque la matrice se trouve à l'état comprimé.

FIG 1

FIG 2

FIG 3

FIG 4

FIG 5

I   II   III   IV   V   VI   VII   VIII   IX   X   XI   XII   XIII   XIV

EP 0 734 749 B1